# EUROPÄISCHE PATENTANMELDUNG

(11) **EP 0 810 264 A2**
(43) Veröffentlichungstag der Anmeldung: **03.12.1997**
(21) Anmeldenummer: 97108002.3
(22) Anmeldetag: 16.05.1997
(51) Int. Cl.: C09B 41/00, C09B 29/15, C07C 245/20

(54) **Verfahren zur Herstellung von Phenylazonaphthalinen**

(30) Priorität: 31.05.1996 DE 19621840
(71) Anmelder: BASF AKTIENGESELLSCHAFT, 67056 Ludwigshafen (DE)
(72) Erfinder: Bermes, Rudolf, Dr., 67069 Ludwigshafen (DE)

(57) **Zusammenfassung**

Verfahren zur Herstellung von wäßrigen Präparationen von Azofarbstoffen der Formel 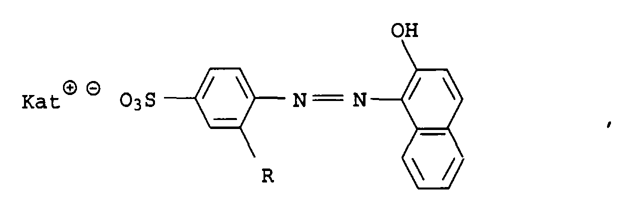 in der
- R: Wasserstoff oder C₁-C₄-Alkyl und
- Kat⊕: das Äquivalent eines Kations bedeutet, das sich von einem tertiären Amin aus der Ethanol- oder Propanolaminreihe ableitet,
durch Diazotierung eines Anilins der Formel 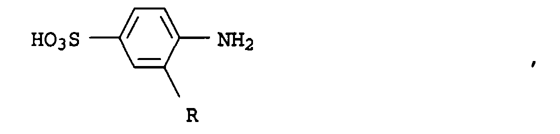 mit Neopentylglykoldinitrit in wäßrigem Medium und Kupplung des resultierenden Reaktionsgemisches mit β-Naphthol in Gegenwart eines mit Wasser mischbaren Verdünnungsmittels und eines Amins, wobei man als Amin ein tertiäres Amin aus der Ethanol- oder Propanolaminreihe verwendet.

## Beschreibung

Die vorliegende Erfindung betrifft ein neues Verfahren zur Herstellung bestimmter Phenylazonaphthaline durch Diazotierung von Anilinsulfonsäuren mittels Neopentylglykoldinitrit in wäßrigem Medium und Kupplung des resultierenden Reaktionsgemisches in Gegenwart eines mit Wasser mischbaren Verdünnungsmittels und eines Amins auf β-Naphthol.

Aus der DE-A- 2 209 478 ist die Herstellung des Farbstoffs C.I. Acid Orange 7 (15 510) ausgehend von 4-Aminobenzolsulfonsäure und β-Naphthol bekannt. Die dort beschriebene Herstellmethode liefert die Farbstofflösungen jedoch in ungenügender Ausbeute. Außerdem enthalten die resultierenden Farbstofflösungen teilweise noch flüchtige Amine.

Die BE-A-889 088 beschreibt die Diazotierung von Sulfanilsäure mit Alkalinitrit und Schwefelsäure in wasserarmem Medium. Die Kupplung mit β-Naphthol erfolgt in Gegenwart von technischem Triethanolamin. Die Ausbeute an so hergestelltem Farbstoff ist jedoch unbefriedigend.

Aufgabe der vorliegenden Erfindung war es daher, ein neues Verfahren zur Herstellung von wäßrigen Präparationen von C.I. Acid Orange 7 und ähnlichen Farbstoffen bereitzustellen, das die obengenannten Nachteile nicht mehr aufweist.

Es wurde nun gefunden, daß die Herstellung von wäßrigen Präparationen von Azofarbstoffen der Formel I 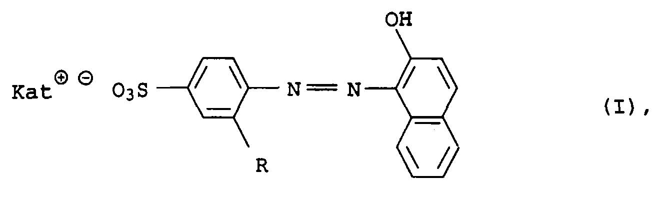 in der
- R: Wasserstoff oder C₁-C₄-Alkyl und
- Kat⊕: das Äquivalent eines Kations bedeutet, das sich von einem tertiären Amin aus der Ethanol- oder Propanolaminreihe ableitet,
durch Diazotierung eines Anilins der Formel II 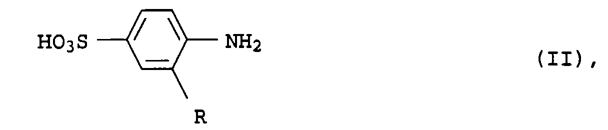 in der R die obengenannte Bedeutung besitzt, mit Neopentylglykoldinitrit in wäßrigem Medium, vorzugsweise in wäßrigem Medium, das im wesentlichen frei ist von organischen Lösungsmitteln, und Kupplung des resultierenden Reaktionsgemisches mit β-Naphthol in Gegenwart eines mit Wasser mischbaren Verdünnungsmittels und eines Amins, vorteilhaft gelingt, wenn man als Amin ein tertiäres Amin aus der Ethanol- oder Propanolaminreihe verwendet.

Reste R sind z.B. Methyl, Ethyl, Propyl, Isopropyl, Butyl, Iso-butyl, sec-Butyl oder tert-Butyl.

Die Herstellung von Farbstoffen der Formel I, in der R Wasserstoff oder Methyl bedeutet, ist bevorzugt, wobei Methyl besonders zu nennen ist.

Geeignete Amine aus der Ethanol- oder Propanolaminreihe sind z.B. N,N-Di-(C₁-C₄-alkyl)ethanol-, -propanol- oder -isopropanolamine, wie N,N-Dimethylethanol-, -propanol- oder -isopropanolamin, N,N-Diethylethanol-, -propanol- oder -isopropanolamin, N,N-Dipropylethanol-, -propanol- oder -isopropanolamin, N,N-Diisopropylethanol-, -propanol- oder -isopropanolamin oder N,N-dibutylethanol-, -propanol- oder -isopropanolamin, N-(C₁-C₄-Alkyl)diethanol-, -dipropanol- oder -diisopropanolamine, wie N-Methyldiethanol-, -dipropanol- oder -diisopropanolamin, N-Ethyldiethanol-, -dipropanol- oder -diisopropanolamin, N-Propyldiethanol-, -dipropanol- oder -diisopropanolamin, N-Isopropyldiethanol-, -dipropanol- oder -diisopropanolamin oder N-Butyldiethanol-, -dipropanol- oder -diisopropanolamin, Triethanolamin, Tripropanolamin, Triisopropanolamin, N-(2-Hydroxyethyl)pyrrolidin, N-(2- oder 3-Hydroxypropyl)pyrrolidin, N-(2-Hydroxylethyl)piperidin, N-(2- oder 3-Hydroxypropyl)piperidin, N-(2-Hydroxyethyl)morpholin, N- (2- oder 3-Hydroxypropyl)morpholin oder N,N-Dimethyl- oder N,N-Diethyl-N-(5-hydroxy-3-oxapentyl)amin.

Besonders geeignete Amine sind Triethanolamin, N-(C₁-C₄-Alkyl)diethanolamine, N,N-Di(C₁-C₄-alkyl)ethanolamine, N,N-Dimethylpropanolamin oder deren Mischungen.

Die Verwendung von N,N-Diethylethanolamin ist besonders hervorzuheben.

Geeignete mit Wasser mischbare Verdünnungsmittel sind z.B. Glykole, wie Propan-1,2-diol, Propan-1,3-diol, Butan-1,2-diol, Butan-2,3-diol, Butan-1,4-diol, Pentan-1,2-diol, Pentan-2,3-diol, Pentan-1,5-diol oder 2,2-Dimethylpropan-1,3-diol (Neopentylglykol), C₁-C₄-Monoalkylether von Mono- oder Diglykolen, wie 2-Methoxyethanol, 2-Ethoxyethanol, 2-Propoxyethanol, 2-Butoxyethanol, 1-Methoxypropan-2-ol, 1-Ethoxypropan-2-ol, 3-Methoxypropanol, 3-Ethoxypropanol, 4-Methoxybutanol, 4-Ethoxybutanol, Diethylenglykolmonomethyl-, -ethyl-, -propyl- oder -butylether oder Dipropylenglykolmonomethyl-, -ethyl-, -propyl- oder -butylether, Di-, Tri- oder Tetraglykole, wie Diethylenglykol, Triethylenglykol, Tetraethylenglykol, Dipropylenglykol, Tripropylenglykol oder Tetrapropylenglykol, cyclische Carbonsäureester, wie γ-Butyrolacton, oder cyclische Carbonsäureamide, wie Pyrrolidon, N-Methyl- oder N-Ethylpyrrolidon oder ε-Caprolactam.

Die Verwendung von Di- oder Triethylenglykol oder Di- oder Tripropylenglykol als wassermischbares Lösungsmittel ist bevorzugt, wobei Dipropylenglykol besonders zu nennen ist.

Je mol Anilin II verwendet man in der Regel 0,5 bis 0,56 mol, vorzugsweise 0,5 bis 0,53 mol, Neopentylglykoldinitrit. Letzteres ist bekannt und wird beispielsweise in der US-A 37 92 077 beschrieben.

Bezogen auf 1 mol Anilin II kommen im allgemeinen 1,0 bis 1,1 mol, vorzugsweise 1,02 bis 1,05 mol, β-Naphthol zur Anwendung.

Bezogen auf 1 mol β-Naphthol verwendet man bei der Kupplung vorzugsweise 1,0 bis 1,15 mol, insbesondere 1,05 bis 1,1 mol, tertiäres Amin aus der Ethanol- oder Propanolaminreihe.

Das β-Naphthol wird vorzugsweise in einer Mischung mit 30 bis 50 Gew.-% Wasser und 45 bis 70 Gew.-% eines mit Wasser mischbaren Verdünnungsmittels jeweils bezogen auf das Gewicht an β-Naphthol in der Kupplungsreaktion eingesetzt.

Die oben genannten Grenzwerte sind nicht bindend. Es können auch andere Gewichtsverhältnisse zur Anwendung gelangen. Von Bedeutung ist dabei, daß das zur Umsetzung gelangende β-Naphthol in Lösung ist.

Die Herstellung des Diazoniumsalzes findet üblicherweise bei einer Temperatur von 5 bis 35°C, vorzugsweise 25 bis 30°C, und bei atmosphärischem Druck statt.

Die Kupplungsreaktion findet im allgemeinen bei einer Temperatur von 5 bis 50°C, vorzugsweise 25 bis 35⁰C, und ebenfalls bei atmosphärischem Druck statt.

Das erfindungsgemäße Verfahren wird zweckmäßig so durchgeführt, daß man in einer geeigneten Apparatur, z.B. in einer Rührapparatur, ein Gemisch aus Wasser und Anilin II vorlegt und bei der obengenannten Temperatur Neopentylglykoldinitrit zudosiert. Nach einer Nachrührzeit von in der Regel 2 bis 4 Stunden ist die Diazotierung beendet.

Unabhängig davon wird in einer anderen Rührapparatur eine Mischung aus Wasser, β-Naphthol, Verdünnungsmittel und tertiärem Amin bereitet und auf die obengenannte Temperatur eingestellt. Danach wird das Reaktionsgemisch aus der Diazotierungsreaktion zudosiert. Nach einer Nachrührphase von in der Regel 15 bis 60 Minuten ist die Umsetzung beendet. Die resultierende Lösung ist, gegebenenfalls nach einer Klärfiltration und der Einstellung auf die gewünschte Farbstärke, direkt gebrauchsfertig.

Sie eignet sich in vorteilhafter Weise zum Färben oder Bedrucken von polymerem Material, insbesondere von Papierstoffen, aber auch von Cellulose, Baumwolle, Leder, Bastfasern, Hanf, Flachs, Sisal, Jute, Kokos oder Stroh.

Das erfindungsgemäße Verfahren liefert die Azofarbstoffe der Formel I in hoher Ausbeute. Sie fallen direkt als wäßrige Lösung, die im neutralen pH-Bereich, d.h. bei einem pH-Wert von ca. 7 bis 8 liegt, an.

Das folgende Beispiel soll die Erfindung näher erläutern.

### Beispiel

a) 177 g 2-Methylanilin-4-sulfonsäure und 9,5 g Anilin-4-sulfonsäure wurden in 600 ml Wasser vorgelegt. Zu dieser Mischung wurden unter Rühren bei 25 bis 30°C innerhalb von 40 min 85,5 g Neopentylglykoldinitrit gegeben. Man rührte 2 h bei 25 bis 30°C nach und zerstörte anschließend überschüssiges Nitrit durch Zugabe von 0,5 g Amidosulfonsäure und kühlte das Reaktionsgemisch auf 10°C ab.
b) Man gab 60 ml Wasser, 85 g Dipropylenglykol, 126 g N,N-Diethylethanolamin und 147 g β-Naphthol in einen Kolben und rührte 1 h. Die resultierende Lösung kühlte man auf 20°C ab und ließ unter Rühren innerhalb von 30 min die unter a) beschriebene Mischung zulaufen. Man gab noch wenig N,N-Diethylethanolamin zum Reaktionsgemisch, bis dessen pH-Wert 7 bis 7,5 betrug. Danach war die Umsetzung beendet.
   Man gab 40 ml Wasser und 3 g Kieselgur hinzu und filtrierte das Reaktionsgemisch über einen Klärfilter. Man erhielt 1430 g einer wäßrigen Lösung, die 432 g des Farbstoffs der Formel 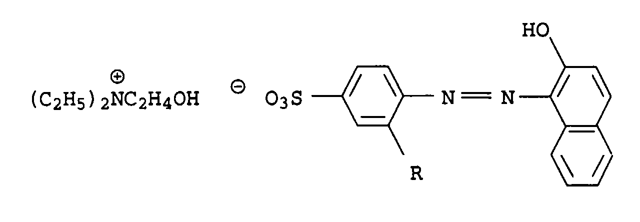 (R = CH₃) und 26 g des Farbstoffs der obengenannten Formel (R = H) enthielt.

### Beispiele 2 bis 5

Man ersetzte das in Beispiel 1b verwendete N,N-Diethylethanolamin durch
- 186 g N,N-Dibutylethanolamin (Beispiel 2)
- 112 g N,N-Dimethylisopropanolamin (Beispiel 3)
- 143 g N,N-Dimethyl-N-(5-hydroxy-3-oxapentyl)amin (Beispiel 4)
- 141 g N-(2-Hydroxyethyl)morpholin (Beispiel 5)
- 174 g N-Butyldiethanolamin (Beispiel 6)
und erhielt jeweils ähnliche günstige Ergebnisse.

### Beispiel 7

a) 173 g 4-Aminobenzolsulfonsäure wurden in 550 ml Wasser vorgelegt und bei 25 bis 30°C unter Rühren mit 85,5 g Neopentylglykoldinitrit versetzt. Man rührte die Mischung bei 28 bis 30°C 2 h nach, zerstörte das verbliebene Nitrit mit 0,8 g Amidosulfonsäure und kühlte die Suspension dabei auf 8°C ab.
b) Man löste 147 g β-Naphthol in einer Mischung aus 85 g Dipropylenglykol, 126 g N,N-Diethylethanolamin und 60 ml Wasser auf. Dann ließ man die unter a) beschriebene Mischung unter Rühren langsam zulaufen, wobei die Temperatur durch Kühlen auf 20 bis 30°C eingestellt wurde. Mit 1,6 g N,N-Diethylethanolamin wurde schließlich der pH-Wert auf 7,4 gebracht. Nach Zugabe von 3 g Kieselgur und 100 ml Wasser lieferte eine Klärfiltration 1334 g einer Lösung, die 445 g des Farbstoffs aus Beispiel 1 (R = H; C.I. Acid Orange 7) enthält.

### Beispiel 8

187 g 2-Methylanilin-4-sulfonsäure wurden analog Beispiel 1a) diazotiert und analog Beispiel 1b) mit β-Naphthol gekuppelt. Man erhielt 1430 g einer Lösung, die 459 g des Farbstoffs aus Beispiel 1 (R = CH₃; C.I. Acid Orange 8) enthält.

### Beispiel 9 bis 15

Man ersetzte das in Beispiel 8 verwendete Dipropylenglykol durch jeweils gleiche Mengen
- Diethylenglykol (Beispiel 9)
- Triethylenglykol (Beispiel 10)
- Butan-1,4-diol (Beispiel 11)
- Pentan-1,5-diol (Beispiel 12)
- Neopentylglykol (Beispiel 13)
- 1-Methoxypropan-2-ol (Beispiel 14)
- Diethylenglykolmonobutylether (Beispiel 15)
und erhielt ähnlich gut konzentrierte Lösungen von C.I. Acid Orange 8.

## Patentansprüche

1. Verfahren zur Herstellung von wäßrigen Präparationen von Azofarbstoffen der Formel I 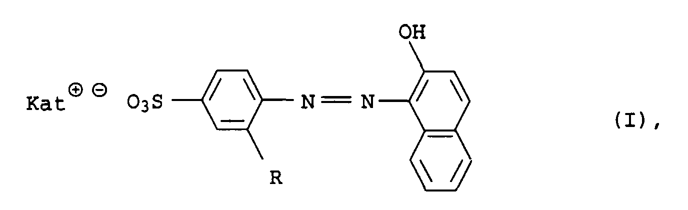 in der
R Wasserstoff oder C₁-C₄-Alkyl und
Kat⊕ das Äquivalent eines Kations bedeutet, das sich von einem tertiären Amin aus der Ethanol- oder Propanolaminreihe ableitet,
durch Diazotierung eines Anilins der Formel II 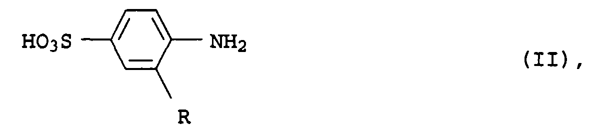 in der R die obengenannte Bedeutung besitzt, mit Neopentylglykoldinitrit in wäßrigem Medium und Kupplung des resultierenden Reaktionsgemisches mit β-Naphthol in Gegenwart eines mit Wasser mischbaren Verdünnungsmittels und eines Amins, dadurch gekennzeichnet, daß man als Amin ein tertiäres Amin aus der Ethanol- oder Propanolaminreihe verwendet.

2. Verfahren nach Anspruch 1, dadurch gekennzeichnet, daß R Wasserstoff oder Methyl bedeutet.

3. Verfahren nach Anspruch 1, dadurch gekennzeichnet, daß man als tertiäres Amin Triethanolamin, N-(C₁-C₄-Alkyl)diethanolamine, N,N-Di(C₁-C₄-alkyl)ethanolamine, N,N-Dimethylpropanolamin oder deren Mischungen verwendet.

4. Verfahren nach Anspruch 1, dadurch gekennzeichnet, daß man als tertiäres Amin N,N-Diethylethanolamin verwendet.
